Europäisches Patentamt

European Patent Office

Office européen des brevets

(11) Publication number: **0 473 105 B1**

# EUROPEAN PATENT SPECIFICATION

(45) Date of publication of patent specification: **01.03.95**  (51) Int. Cl.[6]: **C07C 17/275**, C07C 19/08

(21) Application number: **91114338.6**

(22) Date of filing: **27.08.91**

(54) **Preparation of pentafluorodichloropropanes.**

(30) Priority: **28.08.90 JP 227501/90**
**28.08.90 JP 227502/90**

(43) Date of publication of application:
**04.03.92 Bulletin 92/10**

(45) Publication of the grant of the patent:
**01.03.95 Bulletin 95/09**

(84) Designated Contracting States:
**AT BE CH DE DK ES FR GB IT LI LU NL SE**

(56) References cited:
**US-A- 2 462 402**
**US-A- 3 381 042**
**US-A- 3 795 710**

**CHEMICAL ABSTRACTS, vol. 43, no. 11, 10 June 1949, pages 979, 980 column 4213, Columbus, Ohio, USA; D. D. Coffman et al, "Synthesis of chlorofluoropropanes".**

(73) Proprietor: **DAIKIN INDUSTRIES, LIMITED Umeda Center Building, 4-12 Nakazaki-nishi 2-chome, Kita-ku Osaka-shi, Osaka-fu 530 (JP)**

(72) Inventor: **Aoyama, Hirokazu, c/o Yodogawa Works of Daikin Industries Ltd., 1-1, Nishihitotsuya Settsu-shi, Osaka-fu (JP)**
Inventor: **Kono, Satoru, c/o Yodogawa Works of Daikin Industries Ltd., 1-1, Nishihitotsuya Settsu-shi, Osaka-fu (JP)**
Inventor: **Yasuhara, Takashi, c/o Yodogawa Works of Daikin Industries Ltd., 1-1, Nishihitotsuya Settsu-shi, Osaka-fu (JP)**
Inventor: **Ueda, Souichi, c/o Yodogawa Works of Daikin Industries Ltd., 1-1, Nishihitotsuya Settsu-shi, Osaka-fu (JP)**

Inventor: **Koyama, Satoshi, c/o Yodogawa Works of Daikin Industries Ltd., 1-1, Nishihitotsuya Settsu-shi, Osaka-fu (JP)**

(74) Representative: **Hansen, Bernd, Dr. Dipl.-Chem. et al Hoffmann, Eitle & Partner Patent- und Rechtsanwälte, Postfach 81 04 20 D-81904 München (DE)**

EP 0 473 105 B1

**Description**

The present invention relates to a process for preparing pentafluorodichloropropanes, in particular, 1,1,1,2,2-pentafluoro-3,3-dichloropropane (hereinafter referred to as "R-225ca") and 1,1,2,2,3-pentafluoro-1,3-dichloropropane (hereinafter referred to as "R-225cb") which are substitute compounds for industrially important 1,1,2-trichloro-1,2,2-trifluoroethane and have less influence on the global environment. The present invention also relates to removal of chloroform from R-225ca and/or R-225cb.

Description of the Related Art

Hitherto, R-225ca and R-225 cb are prepared, for example, by batchwise reacting tetrafluoroethylene (hereinafter referred to as "TFE") with dichlorofluoromethane (hereinafter referred to as "R-21") in the presence of a catalyst such as anhydrous aluminum chloride at a temperature of 15 to 100°C in an autoclave or a glass reactor (cf. U.S. Patent No. 2,462,402, J. Am. Chem. Soc., 71, 979 (1949) and Col. Czech. Chem. Com., 36 (1867)).

R-225ca can be prepared by reacting TFE with cesium fluoride in diglyme and further with chloroform (cf. U.S. Patent No. 3,381,042).

In the former process using anhydrous aluminum chloride, a selectivity and a yield of the desired products are low, and the yield is only 46 to 58 %. Then, this process is uneconomical in the industry. After reaction, the products should be separated from the catalyst by collecting the products in a cold trap under reduced pressure or by treating the catalyst with hydrochloric acid and separating the products.

In addition, the products inevitable contain by-products such as chloroform. Since chloroform forms an azeotropic mixture with each of R-225ca and R-225cb, a large amount of R-225ca and R-225cb are lost when chloroform is removed from the mixture by rectification to obtain pure R-225ca or R-225cb.

Though the latter process using cesium fluoride is excellent in the selectivity and yield, cesium fluoride which is one of the starting materials is very expensive. Then, this process is not suitable for the commercial production.

SUMMARY OF THE INVENTION

One object of the present invention is to provide a novel process for preparing R-225ca and R-225cb in a high selectivity and yield.

Another object of the present invention is to provide a process for removing chloroform from R-225ca and/or R-225cb.

According to a first aspect of the present invention, there is provided a process for preparing R-225ca and R-225cb which comprises reacting chloroform, difluorochloromethane and TFE in the presence of a catalyst.

According to a second aspect of the present invention, there is provided a process for removing chloroform from a mixture of chloroform and at least one pentafluorodichloropropane selected from the group consisting of R-225ca and R-225cb, which comprises reacting difluorochloromethane and TFE with chloroform in said mixture in the presence of a catalyst.

DETAILED DESCRIPTION OF THE INVENTION

In either process of the present invention, the catalyst may be any one of catalysts which are catalytically active for an addition reaction of R-21 to TFE. As the catalyst, a Lewis acid is preferred. Examples of the catalyst are anhydrous aluminum chloride, anhydrous titanium tetrachloride, anhydrous tin tetrachloride, anhydrous antimony pentachloride, anhydrous zinc chloride, anhydrous iron chloride, anhydrous aluminum bromide, boron trifluoride, etc. In addition, a compound of the formula:

$$AlCl_xF_yO_z \quad (I)$$

wherein x, y and z are numbers which satisfy the equations:

$x + y + 2z = 3,$
$0 < x < 3,$
$0 \leq y < 3$ and
$0 \leq z < 3/2$

3

provided that at least one of y and z is not zero, such as aluminum chloride fluoride and alumina chloride fluoride can be used.

Aluminum chloride fluoride to be used as a catalyst in the present invention can be prepared by reacting aluminum chloride with hydrogen fluoride, hydrofluoric acid, or a fluoro- or chlorofluoro-hydrocarbon having not more than 4 carbon atoms, preferably not more than 2 carbon atoms (e.g. trifluoromethane, tetrafluoroethane, chlorodifluoromethane, dichlorofluoromethane, trifluorodichloroethane, trifluorochloromethane, difluorotetrachloroethane, trifluorotrichloroethane, etc.). They may be used alone or in combination. Alternatively, they may be used in combination with a chlorohydrocarbon. A reaction temperature is from 0 to 120°C, preferably from 0 to 100°C. The reaction may be carried out in a liquid phase or a vapor phase.

Alumina chloride fluoride to be used as a catalyst in the present invention can be prepared by reacting activated alumina with a chloro-, chlorofluoro- or fluorohydrocarbon, hydrogen chloride or their mixture at a temperature of 100 to 700°C. For example, alumina is filled in a reactor tube made of stainless steel, Hasteloy or glass and heated to a temperature of 300 to 500°C in a stream of dried nitrogen to thoroughly dry alumina. Then, at the above specified temperature, preferably from 100 to 600°C, more preferably from 200 to 400°C, the above halogenated hydrocarbon alone or its mixture with hydrogen chloride or oxygen is passed through the reactor tube. When this temperature is lower than 100°C, the treating time becomes unpractically long, while it is higher than 700°C, carbon will deposit on the alumina surface so that a catalytic activity is deteriorated. The deterioration of catalytic activity can be prevented by the presence of oxygen or air as disclosed in Japanese Patent Publication No. 27375/1986.

Also, alumina chloride fluoride may be prepared by treating activated alumina with hydrogen chloride gas. In a stream of dried nitrogen, activated alumina is heated at a temperature of 400 to 800°C to thoroughly dry alumina. Then, at the above specified temperature, preferably from 300 to 700°C, hydrogen chloride as such or diluted with an inert gas (e.g. nitrogen, argon, etc.) or a chlorofluorohydrocarbon (e.g. R-12 (dichlorodifluoromethane), R-21, etc.) is passed over dried alumina. The passing time is usually from 3 to 10 hours.

As alumina, any commercially available porous alumina which comprises $\gamma$-alumina and is used for dehydration or as a catalyst can be used. Examples of such alumina are Neobead C, MH, GB and D (all manufactured by Mizusawa Chemical Industries, Ltd.) and activated alumina KHA, NKH 1 and NKH 3 (all manufactured by Sumitomo Chemical Company, Ltd.)

As the chloro- or chlorofluoro-hydrocarbon having no hydrogen, those having 1 to 3 carbon atoms, preferably 1 or 2 carbon atoms are used. Preferred examples are carbon tetrachloride, fluorotrichloromethane, difluorodichloromethane, trifluorochloromethane, 1,1,2-trichloro-1,2,2-trifluoroethane, 1,1,1-trichloro-2,2,2-trifluoroethane, 1,1,2,2-tetrafluoro-1,2-dichloroethane, 1,1,1,2-tetrafluoro-2,2-dichloroethane, 1,1,2,2-tetrachloro-1,2-difluoroethane, 1,1,1,2-tetrachloro-2,2-difluoroethane, etc. As the chloro- or chlorofluoro-hydrocarbon having a hydrogen atom, those having 1 to 3 carbon atoms, preferably 1 or 2 carbon atoms are used. Preferred examples are fluorodichloromethane, difluorochloromethane, 1,1,1-trifluoro-2,2-dichloroethane, 1,1,2-trifluoro-1,2-dichloroethane, 1,1,1-trifluoro-2-chloroethane, etc.

Alternatively, the alumina chloride fluoride catalyst can be prepared by reacting alumina with an inorganic fluoride such as hydrogen fluoride at a temperature of 20 to 450°C; sulfur fluoride (e.g. SF$_4$, SF$_6$, etc.), sulfuryl fluoride or thionyl fluoride at a temperature of 300 to 500°C; or an ammonium fluoride (e.g. acidic ammonium fluoride, neutral ammonium fluoride, etc.) at a temperature of 20 to 450°C, and then treating a product with a chlorofluoro- or chloro-hydrocarbon or hydrogen chloride.

The catalysts may be used as a mixture of two or more of them.

Among the catalysts to be used in the present invention, particularly preferred catalyst are anhydrous aluminum chloride and the catalyst of the above formula (I).

In view of economy, the most preferred reaction mode is a continuous reaction mode in which the raw materials are continuously fed and the product is continuously removed, though it is possible to use a semi-batchwise process in which certain amounts of the raw materials are continuously fed and stopped, the reaction is carried out for a certain time and then the reaction product is recovered.

In the process of the present invention for the production of R-225ca and R-225cb, chloroform, R-22 and TFE may be reacted in a solvent.

Any of solvents which are inactive to the catalyst and in which chloroform, R-22 and TFE are dissolved can be used. For example, chloroform may be used as the solvent. Tetrafluorotrichloropropanes which are by-produced hydrochlorofluoroalkanes may be used as the solvents. In addition, a conventional solvent such as chloroalkanes (e.g. dichloromethane) or chlorofluoroalkanes (e.g. tetrachlorotetrafluoropropanes) may be used as the solvent.

4

In view of easy reaction handlings, chloroform which is one of the raw materials is preferred. In view of separation of R-225, R-225 as such is preferred.

When anhydrous aluminum chloride is used as the catalyst and R-225ca, R-225cb or their mixture is used as the solvent, a specific amount of anhydrous aluminum chloride is suspended in the solvent, and then chloroform, R-22 and TFE are charged in the suspension at a specific molar ratio at specific flow rates. As the reaction proceeds, a reaction mixture containing formed R-225ca and R-225cb is separated from suspended anhydrous aluminum chloride and recovered from the reactor. The reaction mixture can be separated from suspended anhydrous aluminum chloride by a conventional method, for example, filtration in a liquid state or distillation in a vapor state. The separated reaction mixture is further purified by a conventional method such as rectification to obtain R-225ca and R-225cb.

A weight ratio of the solvent to the catalyst is at least two. When this ratio is smaller than 2, the reaction system cannot be effectively stirred so that, in an initial stage, the selectivity of R-225ca and R-225cb tends to decrease.

A molar ratio among chloroform, R-22 and TFE will be explained. A molar ratio of chloroform to R-22 is at least 1:1, preferably from 1:1 to 1:10, and a molar ratio of R-22 to TFE is at least 1:2, preferably from 1:2 to 1:10. A molar ratio of chloroform:R-22:TFE is, for example, 1:2:4.

Chloroform, R-22 and TFE may be premixed and then charged in the reactor, or they may be charged simultaneously without premixing. In some cases, a certain amount of chloroform is charged for a certain time and then a mixture of R-22 and TFE is charged.

Each of the raw materials may be used in a gas form or a liquid form.

A reaction pressure is not critical and may be reduced pressure. However, in view of simplicity of the reactor, atmospheric pressure or higher is preferred.

A reaction temperature is usually from -30 to +120°C, preferably from -20 to +60°C. When the reaction temperature is higher than 120°C, amounts of by-products increase and the selectivity of R-225ca and R-225cb decreases. When the reaction temperature is lower than -30°C, the reaction rate becomes unpractically low.

The raw materials, namely chloroform, R-22 and TFE are industrially produced and commercially available. The Lewis acid such as anhydrous aluminum is a commercially available compound and used in the present invention without treatment.

In the process of the present invention for the removal of chloroform, when anhydrous aluminum chloride is used as the catalyst, the catalyst is suspended in the mixture of R-225ca, R-225cb and chloroform, and then TFE and R-22 are charged at the specific ratio at the specific temperature. Progress of the reaction is monitored by gas chromatography and the reaction is terminated when chloroform is not detected.

The suspended catalyst is separated from the mixture to recover desired R-225ca and R-225cb. The separation of the catalyst can be carried in the same way as above.

Since the recovered mixture contains no chloroform, pure R-225ca and R-225cb can be easily and economically obtained by rectification.

In the removal process of the present invention, TFE and R-22 in the gas state are continuously bubbled though the reaction mixture under atmospheric pressure, or the reaction mixture is charged in an autoclave and then TFE and R-22 are charged in the autoclave under pressure to carry out the reaction. A reaction pressure is not critical.

A molar ratio of TFE to R-22 is not limited. At least one mole of TFE is used per one mole of R-22. A large excessive amount of TFE does not have any influence on the reaction. However, unreacted TFE may escape from the reaction mixture and a large amount of TFE is lost. In view of economy, an upper limit of TFE is 3 moles per one mole of R-22.

A reaction temperature may be the same as above.

PREFERRED EMBODIMENTS OF THE INVENTION

The present invention will be explained further in detail by following Examples.

Example 1

To a 100 ml glass flask equipped with a silica gel drying tube for preventing inclusion of moisture in a reaction system and a gas inlet tube, chloroform (15 g) and anhydrous aluminum chloride (1 g) were charged. In a stirred suspension in the flask, TFE and R-22 were premixed at flow rates of 20 cc/min. and 10 cc/min., respectively and charged in the flask through the gas inlet tube, during which the flask was cooled from outside with iced water to keep the reaction temperature in the range between 5°C and 10°C. According to the analysis of the reaction mixture by gas chromatography, it was found that an amount of chloroform decreased while amounts of R-225ca and R-225cb increased as the reaction time passed. After 5 hours reaction, an amount of the reaction mixture was 51 g, and its composition was as follows. No chloroform was detected.

|  | Results of gas chromatography |
|---|---|
| R-225ca | 53.7 % |
| R-225cb | 31.7 % |
| R-224[*)] | 10.8 % |
| Others | 3.8 % |

Note: *) R-224 stands for tetrafluorotrichloropropane.

Example 2

Anhydrous aluminum chloride fluoride was prepared as follows:

Anhydrous aluminum chloride (20 g) and fluorotrichloromethane (20 g) were mixed and stirred at a temperature of 0 to 5°C for 2 hours. Then, the reaction mixture was evaporated under reduced pressure to obtain anhydrous aluminum chloride fluoride.

To the same flask as used in Example 1, the above anhydrous aluminum chloride fluoride (2 g) and R-225 (ca:cb = 51:49) (40 g) were charged. While cooling the flask from outside with iced water and stirring the mixture with a magnetic stirrer, liquid chloroform was charged at a flow rate of 1.33 g/hr. Simultaneously, TFE and R-22 were premixed at flow rates of 20 cc/min. and 10 cc/min., respectively and charged in the flask. After 6 hours reaction, an amount of the reaction mixture was 68 g, and its composition was as follows. No chloroform was detected.

|  | Results of gas chromatography |
|---|---|
| R-225ca | 57.7 % |
| R-225cb | 36.7 % |
| R-224[*)] | 2.3 % |
| Others | 3.3 |

Note: *) R-224 stands for tetrafluorotrichloropropane.

Example 3

In a 100 ml three-necked flask equipped with a silica gel drying tube, a mixture of R-225ca and R-225 (ca:cb = 92:8) (40 g) containing 3 % by mole of chloroform was charged. After charging anhydrous aluminum chloride (2 g), R-22 and TFE were premixed at flow rates of 10 cc/min. and 30 cc/min., respectively and charged in the flask while stirring with a magnetic stirrer and cooling the flask from outside to keep the internal temperature at 5°C. After 6 hours, a concentration of chloroform in the mixture of R-225ca and R-225cb was reduced to 0.9 % by mole.

Example 4

In the same manner as in Example 2, anhydrous aluminum chloride fluoride was prepared.

In a 500 ml three-necked flask equipped with a silica gel drying tube, a mixture of R-225ca and R-225 (ca:cb = 92:8) (400 g) containing 3 % by mole of chloroform was charged. After charging above anhydrous aluminum chloride (8 g), R-22 and TFE were premixed at flow rates of 40 cc/min. and 40 cc/min., respectively and charged in the flask while stirring with a magnetic stirrer and cooling the flask from outside to keep the internal temperature at 5°C. After 5 hours, a concentration of chloroform in the mixture of R-225ca and R-225cb was reduced to 0.3 % by mole.

Example 5

In a 300 ml autoclave made of SUS 316, a mixture of R-225ca and R-225cb (ca:cb = 52:48) (80 g) containing 1.2 % by mole of chloroform and anhydrous aluminum chloride (4 g) were charged, and after cooling the autoclave, air in the autoclave was removed under reduced pressure. Then, R-22 (1.5 g) and TFE (3.3 g) were charged in a gas state and the mixture was stirred at 25°C for 3 hours. The liquid in the autoclave was analyzed by gas chromatography to find that a concentration of chloroform in the mixture of R-225ca and R-225cb was reduced to 0.1 % by mole.

Example 6

In a 100 ml reactor equipped with an ice-cooled condenser an exit of which was connected with a trap cooled at -70°C, R-225ca (20 g) and ground anhydrous aluminum chloride (1 g) were charged. The mixture was stirred to suspend anhydrous aluminum chloride in R-225ca. Then, R-21 and TFE were premixed at flow rates of 10 cc/min. and 30 cc/min., respectively and charged in the suspension in the reactor to start a reaction. After 30 minutes, a temperature of the reaction mixture rose to 40°C. After continuously charging R-21 and TFE for 4 hours, the supply of R-21 and TFE was stopped, and aluminum chloride was filtrated off from the liquid in the reactor. The filtrate was analyzed by gas chromatography. The results are as follows:

|  | Results of gas chromatography |
| --- | --- |
| TFE | 0.9 % |
| R-21 | 0.1 % |
| R-225ca,cb | 94.5 % |
| CHCl$_3$ | 2.0 % |
| R-225 | 2.5 |

Weight: 33.5 g.

In a 100 ml three-necked flask equipped with a silica gel drying tube, this mixture of R-225ca and R-225 (ca:cb = 52:48) (30 g) containing 2 % by mole of chloroform was charged. After charging anhydrous aluminum chloride (1.5 g), R-22 and TFE were premixed at flow rates of 10 cc/min. and 10 cc/min., respectively and charged in the flask while stirring with a magnetic stirrer and cooling the flask from outside to keep the internal temperature at 5°C. After 6 hours, a concentration of chloroform in the mixture of R-225ca and R-225cb was reduced to 0.1 % by mole.

**Claims**

1.  A process for preparing 1,1,1,2,2-pentafluoro-3,3-dichloropropane and 1,1,2,2,3-pentafluoro-1,3-dichloropropane which comprises reacting chloroform, difluorochloromethane and tetrafluoroethylene in the presence of a catalyst.

2.  The process according to claim 1, wherein said catalyst is a Lewis acid.

3.  The process according to claim 1, wherein said catalyst is at least one selected from the group consisting of anhydrous aluminum chloride, anhydrous titanium tetrachloride, anhydrous tin tetrachloride, anhydrous antimony pentachloride, anhydrous zinc chloride, anhydrous iron chloride, anhydrous aluminum bromide, boron trifluoride, and a compound of the formula:

$AlCl_xF_yO_z$      (I)

wherein x, y and z are numbers which satisfy the equations:

$x + y + 2z = 3,$

$0 < x < 3,$
$0 \leqq y < 3$ and
$0 \leqq z < 3/2$

provided that at least one of y and z is not zero.

4.  The process according to claim 1, wherein the reaction is carried out in the absence of an additional solvent.

5.  The process according to claim 1, wherein a reaction temperature is from -30 to +120°C.

6.  A process for removing chloroform from a mixture of chloroform and at least one pentafluorodichloropropane selected from the group consisting of 1,1,1,2,2-pentafluoro-3,3-dichloropropaneand 1,1,2,2,3-pentafluoro-1,3-dichloropropane, which comprises reacting difluorochloromethane and tetrafluoroethylene with chloroform in said mixture in the presence of a catalyst.

7.  The process according to claim 6, wherein said catalyst is a Lewis acid.

8.  The process according to claim 6, wherein said catalyst is at least one selected from the group consisting of anhydrous aluminum chloride, anhydrous titanium tetrachloride, anhydrous tin tetrachloride, anhydrous antimony pentachloride, anhydrous zinc chloride, anhydrous iron chloride, anhydrous aluminum bromide, boron trifluoride, and a compound of the formula:

$$AlCl_xF_yO_z \qquad (I)$$

wherein x, y and z are numbers which satisfy the equations:

$x + y + 2z = 3,$
$0 < x < 3,$
$0 \leqq y < 3$ and
$0 \leqq z < 3/2$

provided that at least one of y and z is not zero.

**Patentansprüche**

1.  Verfahren zur Herstellung von 1,1,1,2,2,-Pentafluor-3,3-dichlorpropan und 1,1,2,2,3,-Pentafluor-1,3-dichlorpropan, umfassend das Umsetzen von Chloroform, Difluorchlormethan und Tetrafluorethylen in Gegenwart eines Katalysators.

2.  Verfahren gemäß Anspruch 1, bei dem der Katalysator eine Lewissäure ist.

3.  Verfahren gemäß Anspruch 1, bei dem der Katalysator wenigstens einer ausgewählt aus der Gruppe, bestehend aus wasserfreiem Aluminiumchlorid, wasserfreiem Titantetrachlorid, wasserfreiem Zinntetrachlorid, wasserfreiem Antimonpentachlorid, wasserfreiem Zinkchlorid, wasserfreiem Eisenchlorid, wasserfreiem Aluminiumbromid, Bortrifluorid und einer Verbindung der Formel:

$$AlCl_xF_yO_2 \qquad (I)$$

worin x, y und z Zahlen sind, welche den Gleichungen entsprechen:

$x + y + 2z = 3,$
$0 < x < 3,$
$0 \leqq y < 3$ und
$0 \leqq z < 3/2$

unter der Voraussetzung, daß wenigstens eines von y und z nicht 0 ist, ist.

**4.** Verfahren gemäß Anspruch 1, bei dem die Umsetzung in Abwesentheit eines zusätzlichen Lösungsmittels durchgeführt wird.

**5.** Verfahren gemäß Anspruch 1, bei dem die Umsetzungstemperatur von -30 bis +120°C ist.

**6.** Verfahren zum Entfernen von Chloroform aus einer Mischung von Chloroform und wenigstens einem Pentafluordichlorpropan ausgewählt aus der Gruppe, bestehend aus 1,1,1,2,2,-Pentafluor-3,3-dichlorpropan und 1,1,2,2,3,-Pentafluor-1,3-dichlorpropan, umfassend das Umsetzen von Difluorchlormethan und Tetrafluorethylen mit Chloroform in der Mischung in Gegenwart eines Katalysators.

**7.** Verfahren gemäß Anspruch 6, bei dem der Katalysator eine Lewissäure ist.

**8.** Verfahren gemäß Anspruch 6, bei dem der Katalysator wenigstens einer ausgewählt aus der Gruppe, bestehend aus wasserfreiem Aluminiumchlorid, wasserfreiem Titantetrachlorid, wasserfreiem Zinntetrachlorid, wasserfreiem Antimonpentachlorid, wasserfreiem Zinkchlorid, wasserfreiem Eisenchlorid, wasserfreiem Aluminiumbromid, Bortrifluorid und einer Verbindung der Formel:

$$AlCl_xF_yO_2 \qquad (I)$$

worin x, y und z Zahlen sind, welche den Gleichungen entsprechen:

$$x + y + 2z = 3,$$
$$0 < x < 3,$$
$$0 \leqq y < 3 \text{ und}$$
$$0 \leqq z < 3/2$$

unter der Voraussetzung, daß wenigstens eines von y und z nicht 0 ist, ist.

**Revendications**

**1.** Procédé de préparation de 1,1,1,2,2-pentafluoro-3,3-dichloropropane et de 1,1,2,2,3-pentafluoro-1,3-dichloropropane qui comprend la réaction de chloroforme, de difluorochlorométhane et de tétrafluoroéthylène en présence d'un catalyseur.

**2.** Procédé selon la revendication 1, dans lequel ledit catalyseur est un acide de Lewis.

**3.** Procédé selon la revendication 1, dans lequel ledit catalyseur est au moins un catalyseur choisi dans le groupe constitué de chlorure d'aluminium anhydre, de tétrachlorure de titane anhydre, de tétrachlorure d'étain anhydre, de pentachlorure d'antimoine anhydre, de chlorure de zinc anhydre, de chlorure de fer anhydre, de bromure d'aluminium anhydre, de trifluorure de bore et d'un composé de formule :

$$AlCl_xF_yO_z \qquad (I)$$

dans laquelle x, y et z sont des nombres qui satisfont aux équations :

$$x + y + 2z = 3,$$
$$0 < x < 3,$$
$$0 \leqq y < 3 \text{ et}$$
$$0 \leqq z < 3/2$$

étant donné qu'au moins un des nombres y ou z est différent de zéro.

**4.** Procédé selon la revendication 1, dans lequel la réaction est mise en oeuvre en l'absence d'un solvant supplémentaire.

**5.** Procédé selon la revendication 1, dans lequel la température de réaction est comprise entre -30 et +120°C.

**6.** Procédé d'élimination de chloroforme d'un mélange de chloroforme et d'au moins un pentafluorodichloropropane choisi dans le groupe constitué de 1,1,1,2,2-pentafluoro-3,3-dichloropropane et de 1,1,2,2,3-pentafluoro-1,3-dichloropropane, qui comprend la réaction de difluorochlorométhane et de tétra-fluoroéthylène avec du chloroforme dans ledit mélange en présence d'un catalyseur.

**7.** Procédé selon la revendication 6, dans lequel ledit catalyseur est un acide de Lewis.

**8.** Procédé selon la revendication 6, dans lequel ledit catalyseur est au moins un catalyseur choisi dans le groupe constitué de chlorure d'aluminium anhydre, de tétrachlorure de titane anhydre, de tétrachlorure d'étain anhydre, de pentachlorure d'antimoine anhydre, de chlorure de zinc anhydre, de chlorure de fer anhydre, de bromure d'aluminium anhydre, de trifluorure de bore et d'un composé de formule :

$$AlCl_xF_yO_z \qquad (I)$$

dans laquelle x, y et z sont des nombres qui satisfont aux équations :

$$x + y + 2z = 3,$$
$$0 < x < 3,$$
$$0 \leq y < 3 \text{ et}$$
$$0 \leq z < 3/2$$

étant donné qu'au moins un des nombres y ou z est différent de zéro.